Europäisches Patentamt

European Patent Office

Office européen des brevets

(19)

(11) Veröffentlichungsnummer: **0 152 896**
**A2**

# (12) EUROPÄISCHE PATENTANMELDUNG

(21) Anmeldenummer: **85101476.1**

(22) Anmeldetag: **12.02.85**

(51) Int. Cl.⁴: **A 61 K 31/545, A 61 K 9/02**

(30) Priorität: **22.02.84 CH 858/84**

(43) Veröffentlichungstag der Anmeldung: **28.08.85**
**Patentblatt 85/35**

(84) Benannte Vertragsstaaten: **AT BE CH DE FR GB IT LI LU NL SE**

(71) Anmelder: **F. HOFFMANN-LA ROCHE & CO.**
**Aktiengesellschaft, CH-4002 Basel (CH)**

(72) Erfinder: **Füller, Walter, Dr., Pappelstrasse 5,**
**CH-4106 Therwil (CH)**

(74) Vertreter: **Lederer, Franz, Dr. et al, Patentanwälte Dr.**
**Lederer Franz Meyer-Roxlau Reiner F.**
**Lucile-Grahn-Strasse 22, D-8000 München 80 (DE)**

(54) Suppositorien enthaltend Ceftriaxon.

(57) Es werden Suppositorien, enthaltend als Wirkstoff eine therapeutisch wirksame Menge an Ceftriaxon, einem pharmazeutisch verträglichen Salz davon, oder einem Hydrat einer dieser Verbindungen, einen Stabilisator, bestehend aus einem Mono-, Di- oder Triglycerid einer $C_{12}$–$C_{18}$-Fettsäure oder aus einem Gemisch solcher Glyceride, einen Potentiator, bestehend aus einer aliphatischen $C_2$–$C_{18}$-Fettsäure, einem Mono-, Di- oder Triglycerid einer $C_2$–$C_{12}$-Fettsäure, einem partiellen Ester oder Vollester von Propylenglykol, Polyäthylenglykol oder eines Kohlenhydrats mit einer $C_2$–$C_{12}$-Fettsäure, einem pharmazeutischen verträglichen Ester oder Äther davon oder aus einem Gemisch der erwähnten Potentiatoren, und erwünschtenfalls herkömmliche therapeutisch inerte Hilfsstoffe für Suppositorien, und deren Herstellung beschrieben. Diese besitzen wertvolle antimikrobielle Eigenschaften.

F. HOFFMANN-LA ROCHE & CO. Aktiengesellschaft, Basel/Schweiz

**0152896**

RAN 4410/190

## Suppositorien enthaltend Ceftriaxon

Die vorliegende Erfindung betrifft Suppositorien, enthaltend als Wirkstoff eine therapeutisch wirksame Menge an Ceftriaxon, einem pharmazeutisch verträglichen Salz davon, oder einem Hydrat einer dieser Verbindungen, einen Stabilisator, bestehend aus einem Mono-, Di- oder Triglycerid einer $C_{12}$-$C_{18}$-Fettsäure oder aus einem Gemisch solcher Glyceride, einen Potentiator, bestehend aus einer aliphatischen $C_2$-$C_{18}$-Fettsäure, einem Mono-, Di- oder Triglycerid einer $C_2$-$C_{12}$-Fettsäure, einem partiellen Ester oder Vollester von Propylenglykol, Polyäthylenglykol oder eines Kohlenhydrats mit einer $C_2$-$C_{12}$-Fettsäure, einem pharmazeutischen verträglichen Ester oder Aether davon oder aus einem Gemisch der erwähnten Potentiatoren, und erwünschtenfalls herkömmliche, therapeutisch inerte Hilfsstoffe für Suppositorien, und ein Verfahren zu deren Herstellung. Diese Suppositorien besitzen wertvolle antimikrobielle Eigenschaften.

Ceftriaxon, seine pharmazeutisch verträglichen Salze und die Hydrate dieser Verbindungen (nachstehend als Wirkstoffe bezeichnet) sind relativ instabile Verbindungen, welche bei

Nt/21.11.84

längerer Lagerung, insbesondere bei Raumtemperatur und Temperaturen darüber, in unwirksame Zersetzungsprodukte übergehen. Bei der Herstellung von pharmazeutischen Zubereitungsformen stellt diese thermische Labilität ein schwierig zu überwindendes Hindernis dar.

Es hat sich nun überraschend herausgestellt, dass die Kombination der Wirkstoffe mit einem Mono-, Di- oder Triglycerid einer $C_{12}$-$C_{18}$-Fettsäure oder einem Gemisch solcher Glyceride eine beträchtliche Erhöhung der Stabilität und Lagerfähigkeit der Wirkstoffe bewirkt. Dies ist umso vorteilhafter, als die Kombination der Wirkstoffe mit dem oben definierten Potentiator oft eine unerwünschte Herabsetzung der Stabilität des Wirkstoffes bewirkt. Dies ist beispielsweise der Fall bei Verwendung von CAPMUL MCM 90 (Gemisch von Mono- und Diglyceriden gesättigter $C_8/C_{10}$-Fettsäuren mit 90% Monoglycerid; Stokely-Van Camp Inc. Columbus, Ohio, USA) oder von CAPMUL 8210 (ähnliche Zusammensetzung wie CAPMUL MCM 90, jedoch mit etwa 70% Monoglyceriden anstelle von 90%) als Potentiator.

Die Wirkstoffe werden rektal an sich nur wenig resorbiert. Zur Erhöhung der Resorption wird deshalb der oben definierte Potentiator eingesetzt.

Die beschriebene Dreierkombination führt zu stabilen Präparaten, aus welchen die Wirkstoffe nach rektaler Verabreichung zudem noch besser resorbiert werden und stellt demgemäss eine wertvolle Bereicherung des Arzneimittelschatzes dar.

Als Wirkstoff verwendet man vorzugsweise Ceftriaxon--Dinatriumsalz-Trihydrat. Bevorzugte Stabilisatoren sind Gemische von Mono-, Di- und/oder Triglyceriden von aliphatischen $C_{12}$-$C_{18}$-Fettsäuren. Bevorzugte Potentiatoren sind gesättigte $C_6$-$C_{12}$-Fettsäuren, ungesättigte $C_{16}$-$C_{18}$--Fettsäuren, Mono-, Di- oder Triglyceride von $C_8$-$C_{12}$-

-Fettsäuren oder Mischungen davon, diese enthaltende Speiseöle und Mischungen davon. Besonders bevorzugte Potentiatoren
sind Mischungen, welche vorwiegend Mono- und Diglyceride von
$C_8-C_{10}$-Fettsäuren, insbesondere von Capryl- und Caprinsäure, enthalten. Sie weisen vorzugsweise einen überwiegenden Anteil an Monoglycerid auf. Der Anteil an Monoglycerid
liegt vorzugsweise in einem Bereich von etwa 70% bis etwa
90%.

Der Stabilisator ist z.B. ein Mono-, Di- oder Triglycerid einer geradkettigen oder verzweigtkettigen, gesättigten oder ungesättigten $C_{12}-C_{18}$-Fettsäure. Beispiele
solcher Fettsäuren sind Laurinsäure, Myristinsäure, Palmitinsäure, Stearinsäure, Lauroleinsäure ($\Delta 9$-Dodecylen-
säure), Palmitoleinsäure, Oelsäure, Ricinolsäure, Linolsäure
und Linolensäure. Als Stabilisator verwendet man vorzugsweise ein Gemisch aus Mono-, Di- und/oder Triglyceriden von
$C_{12}-C_{18}$-Fettsäuren, das vorwiegend aus dem Triglycerid
der Laurinsäure ($C_{12}$) besteht.

Der Potentiator ist z.B. eine geradkettige oder verzweigtkettige, gesättigte oder ungesättigte $C_2-C_{18}$-Fett-
säure. Beispiele solcher Fettsäuren sind Buttersäure,
Capronsäure, Caprylsäure, Caprinsäure, Laurinsäure, Myristinsäure, Palmitinsäure, Stearinsäure, Lauroleinsäure ($\Delta 9$-
-Dodecylensäure), Palmitoleinsäure, Oelsäure, Ricinolsäure,
Linolsäure und Linolensäure.

Unter "Fettsäuren" sollen gesättigte oder ungesättigte,
monobasische aliphatische Carbonsäuren verstanden werden,
welche mit Glycerin oder anderen Alkoholen Ester bilden,
wobei Fette, Oele, Wachse und andere Lipide entstehen.

Der Ausdruck "Glyceride" bezieht sich auf Ester von
Glyerin, einschliesslich der Fette und Oele, worin bis zu
drei Moleküle Fettsäure an einem Molekül Glycerin gebunden
sind. Obwohl mehrheitlich Glyceride mit gleicher Fettsäure verwendet werden, kommen auch Glyceride mit verschiedenen Fettsäureresten in Frage. Bei Verwendung von Glyceriden mit verschiedenen Fettsäureresten können optisch aktive Verbindungen auftreten.

Als Potentiatoren kommen auch die pharmazeutisch verträglichen Ester und Aether der erwähnten Mono- und Diglyceride und der erwähnten partiellen Ester in Frage.

Zur Herstellung der erwähnten Ester geeignete Veresterungsagenzien sind beispielsweise solche, die sich von pharmazeutisch verträglichen schwachen Säuren, wie Weinsäure und ihren Diacetylderivaten, Essigsäure, Ascorbinsäure, Zitronensäure und Phosphorsäuren mit einer Monophosphatgruppe, die einen Monophosphatester bilden können, ableiten.

Geeignete Aether können gebildet werden durch Verätherung der freien Hydroxylgruppe(n) mit einer reaktionsfähigen niederen Alkyl-, Alkenyl-, Alkinyl-, Aryl- oder substituierten Arylverbindung, wobei der entsprechende, pharmazeutisch verträgliche Aether entsteht. Diese Umsetzung kann in an sich bekannter Weise erfolgen.

Die erfindungsgemässen Suppositorien enthalten vorzugsweise etwa 25 mg bis etwa 2000 mg Ceftriaxon, insbesondere etwa 50 mg bis etwa 500 mg.

Das Verhältnis Wirkstoff:Stabilisator in den erfindungsgemässen Suppositorien variiert zweckmässigerweise zwischen etwa 1:32 und etwa 1:1 und liegt vorzugsweise zwischen etwa 1:5 und etwa 1:1,5. Das Verhältnis Wirkstoff:Potentiator liegt zweckmässigerweise zwischen etwa 1:32 und etwa 1:0,2, vorzugsweise zwischen etwa 1:16 und etwa 1:0,5.

Die erfindungsgemässen Suppositorien können auch an sich bekannte Hilfsstoffe zwecks Erreichung einer gewünschten Konsistenz enthalten. Sie können beispielsweise wasserlösliche Träger, wie Polyäthylenglykol, Polypropylenglykol, Glycerogelatine, Methylcellulose oder Carboxymethylcellulose, enthalten. Netzmittel kommen ebenfalls in Betracht, z.B. nichtionische Netzmittel, wie Polyoxyäthylensorbitan--Fettsäureester, Polyoxyäthylensorbitol-Fettsäureester, Polyoxyäthylen-Fettsäureester, Glycerin-Fettsäureester sowie höhere Alkoholester von Polyoxyäthylen, oder anionische Netzmittel, wie Ester von niederen Alkylsulfonsäuren. Die Suppositorien können ferner geeignete Emulgier- und Dispergiermittel, Mittel zur Einstellung der Viskosität und Farbstoffe enthalten.

Diese Suppositorien können erfindungsgemäss dadurch hergestellt werden, dass man den Stabilisator zusammen mit dem Potentiator durch Erwärmen schmilzt, den Wirkstoff und erwünschtenfalls herkömmliche, therapeutisch inerte Hilfsstoffe für Suppostorien in der erhaltenen Schmelze gleichmässig dispergiert und die erhaltene Dispersion mittels Suppositorienformen entsprechender Grösse zu Suppositorien formuliert.

Zum Nachweis der erhöhten Stabilität der erfindungsgemässen Suppositorien im Vergleich zu Suppositorien ohne Stabilisator wurde das weiter unten beschriebene Suppositorium A mit dem nachstehend beschriebenen Suppositorium X verglichen:

Suppositorium X

| | | |
|---|---|---|
| Ceftriaxon-Dinatriumsalz-Trihydrat | 596,5 | mg |
| (entspricht 500 mg Ceftriaxon) | | |
| CAPMUL MCM 90 | 500 | mg |
| Gelatine (Pulver) | 160 | mg |
| D-Mannit (Pulver) | 25 | mg |
| | 1281,5 | mg |

- 6 -                    0152896

Dieses Suppositorium wird analog Suppositorium A angefertigt und anschliessend lyophilisiert.

Versuch A:

Nach 10-monatiger Lagerung unter gleichen Bedingungen
werden die Suppositorien A und X wie folgt auf Stabilität
untersucht:

Von jedem Suppositorium wird ein bestimmter Teil abgeschnitten. Diese Teile werden bei 40°C jeweils in 3 ml
100-proz. Dimethylsulfoxid gelöst. Nach Verdünnen mit bestimmten Mengen an Wasser (entsprechend der Standardkurve
für Ceftriaxon; 0,125 µg/ml-4 µg/ml) werden die Proben
anhand mikrobiologischer "Grossplattenanalyse" gemäss
Analyst 80, 95, 110, 531 (1955) untersucht. Als Mikroorganismus wird E. coli 1346 verwendet. Die Menge an Ceftriaxon
in den abgeschnittenen Teilen wird anhand der Standardkurve
ermittelt. Die Ergebnisse sind in der Tabelle I zusammengestellt.

Tabelle I

|  | Menge an Ceftriaxon in den verschiedenen Teilen | | | |
|  | theoretisch in mg | gefunden in mg | in % | Durchschnitt in % |
| --- | --- | --- | --- | --- |
| Suppositorium A | 24,8 | 25,4 | 102 | 98,8 |
|  | 24,8 | 23,7 | 95,6 |  |
| Suppositorium X | 64,3 | 54,4 | 84,6 | 77,4 |
|  | 64,3 | 45,2 | 70,3 |  |

Versuch B:

Nach 12-monatiger Lagerung bei 5°C werden die Suppositorien A und X wie folgt mittels HPLC-Analyse auf Stabilität untersucht:

Ein Teil des Suppositoriums A wird unter leichtem Erwärmen in 1,0 ml Dimethylsulfoxid gelöst. Man verdünnt mit der frisch filtrierten mobilen Phase, bestehend aus 4,0 g Tetraheptylammoniumbromid, 500 ml Acetonitril, 440 ml Wasser, 55 ml Phosphatpuffer vom pH 7,0 und 5 ml Citratpuffer vom pH 5,0, auf 50 ml, filtriert durch einen Miller-HV-Filter (0,45 μm) und analysiert die erhaltene Lösung mittels HPLC.

Das Suppositorium X wird in 50 ml der obigen mobilen Phase gelöst, und die erhaltene Lösung wird wie oben filtriert und mittels HPLC analysiert.

Die erhaltenen Resultate sind in der nachfolgenden Tabelle II zusammengestellt.

<u>Tabelle II</u>

|  | Ermittelte Menge an Ceftriaxon in % |
|---|---|
| Suppositorium A | 94 |
| Suppositorium X | 51 |

Der nachfolgende Vergleich der Wirksamkeit der erfindungsgemässen Suppositorien mit herkömmlichen Suppositorien wurde anhand einmaliger rektaler Verabreichung von Suppositorien an Humanprobanden getestet. Zur Verwendung gelangten das bereits erwähnte Suppositorium A und ein Suppositorium Y, das mit Suppositorium X identisch ist, jedoch jeweils

0152896

300 mg Gelatine anstatt 160 mg enthält. Diese Suppositorien wurden den gleichen Probanden nacheinander verabreicht. Die Plasmaspiegel an Ceftriaxon wurden durch Analysen von Blutproben bestimmt, wobei die in J. High Resolution Chromatographic Comm. No. 4, 1981, S. 54-59 angegebene Analysenmethode verwendet wurde. Die Ergebnisse sind in der Tabelle III zusammengestellt. Diese zeigen deutlich, dass mit dem erfindungsgemässen Suppositorium A regelmässig höhere Blutspiegel an Ceftriaxon im Blut erreicht werden als mit dem Suppositorium Y, das keinen Stabilisator enthält.

## Tabelle III

Konzentration an Ceftriaxon in µg/ml im Plasma von Humanprobanden nach einmaliger rektaler Verabreichung von 500 mg Ceftriaxon

| Proband | Suppositorium | 15 min. | 30 min. | 1 Std. | 1,5 Std. | 2 Std. | 3 Std. | 4 Std. | 5 Std. | 6 Std. | 8 Std. | 10 Std. | 24 Std. |
|---|---|---|---|---|---|---|---|---|---|---|---|---|---|
| 1 | A | 3,78 | 12,53 | 11,54 | 10,37 | 9,34 | 8,61 | 7,22 | 5,93 | 4,41 | 4,27 | 3,44 | 1,04 |
|   | Y | n.m.* | n.m. | n.m. | n.m. | n.m. | n.m. | n.m. | n.m. | n.m. | n.m. | n.m. | n.m. |
| 2 | A | 6,24 | 9,18 | 9,10 | 8,36 | 7,28 | 6,20 | 5,07 | 4,19 | 3,44 | 2,82 | 1,30 | n.m. |
|   | Y | 1,96 | 3,78 | 5,28 | 4,19 | 3,95 | 3,28 | 2,83 | 2,44 | 2,03 | 1,54 | 1,32 | n.m. |
| 3 | A | 1,71 | 3,65 | 4,97 | 4,27 | 3,83 | 3,06 | 2,71 | 2,39 | 1,83 | 1,67 | 1,39 | n.m. |
|   | Y | 2,06 | 6,09 | 4,66 | 4,57 | 4,30 | 3,41 | 3,23 | - | 2,15 | 1,88 | 1,34 | n.m. |
| 4 | A | 7,38 | 10,36 | 11,15 | 10,08 | 8,90 | 8,31 | 7,34 | 6,25 | 5,35 | 5,31 | 4,11 | 1,70 |
|   | Y | 2,64 | 3,71 | 3,77 | 3,56 | 3,34 | 3,20 | 2,98 | 2,52 | 2,30 | 2,18 | 1,94 | 0,49 |
| 5 | A | 8,20 | 13,15 | 11,58 | 10,29 | 9,63 | - | 8,23 | 7,39 | 6,05 | 5,76 | 5,04 | 1,63 |
|   | Y | 0,96 | 4,05 | 4,60 | 4,97 | 4,83 | 4,05 | 3,56 | 3,17 | 2,94 | 2,61 | 2,42 | 0,94 |
| 6 | A | 3,20 | 9,70 | 9,80 | 9,10 | 8,90 | 7,90 | 5,90 | 5,70 | 3,70 | 3,40 | 3,00 | n.m. |
|   | Y | 3,46 | 5,21 | 8,89 | 8,76 | 8,07 | 7,04 | 6,15 | 5,41 | 4,89 | 4,05 | 3,41 | 0,96 |

* n.m. = nicht messbar

Das folgende Beispiel dient der näheren Erläuterung der vorliegenden Erfindung.

## Beispiel

Suppositorium A:

| | |
|---|---|
| Ceftriaxon-Dinatriumsalz-Trihydrat (entspricht 500 mg Ceftriaxon) | 596,5 mg |
| CAPMUL MCM 90 (Gemisch von Mono- und Diglyceriden gesättigter $C_8/C_{10}$-Fettsäuren mit 90% Monoglycerid; Stokely-Van Camp. Inc. Columbus, Ohio, USA) | 500 mg |
| Witepsol H15 (Gemisch von Mono-, Di- und Triglyceriden gesättigter $C_{12}$-$C_{18}$-Fettsäuren; Dynamit Nobel A.G., Deutschland) | 1303,5 mg |
| | 2400,0 mg |

Das Witepsol wird mit dem CAPMUL auf 50°C erwärmt und geschmolzen. Die Schmelze wird mit dem Wirkstoff unter Rühren vermischt, um eine gleichmässige Dispersion in der Grundmasse zu erhalten. Die erhaltene Dispersion wird zu Suppositorien gegossen, die das oben angegebene Gewicht aufweisen.

In analoger Weise können Suppositorien mit der in der Tabelle IV angegebenen Zusammensetzung hergestellt werden.

- 11 -

0152896

## Tabelle IV

|  | Menge in mg an | | | Gewicht in |
|---|---|---|---|---|
|  | Ceftriaxon* | CAPMUL MCM 90 | Witepsol H15 | mg des Suppositoriums |
| Suppositorium B | 200 | 500 | 3300 | 4000 |
| " C | 150 | 500 | 3350 | 4000 |
| " D | 300 | 500 | 3200 | 4000 |
| " E | 600 | 500 | 2900 | 4000 |
| " F | 1200 | 500 | 2300 | 4000 |
| " G | 600 | 1750 | 1650 | 4000 |
| " H | 250 | 250 | 500 | 1000 |
| " I | 500 | 500 | 1000 | 2000 |
| " J | 1000 | 1000 | 2000 | 4000 |

* in Form von Ceftriaxon-Dinatriumsalz-Trihydrat

0152896

## Patentansprüche

1. Suppositorien, enthaltend als Wirkstoff eine therapeutisch wirksame Menge an Ceftriaxon, einem pharmazeutisch verträglichen Salz davon, oder einem Hydrat einer dieser Verbindungen, einen Stabilisator, bestehend aus einem Mono-, Di- oder Triglycerid einer $C_{12}$-$C_{18}$-Fettsäure oder aus einem Gemisch solcher Glyceride, einen Potentiator, bestehend aus einer aliphatischen $C_2$-$C_{18}$-Fettsäure, einem Mono-, Di- oder Triglycerid einer $C_2$-$C_{12}$-Fettsäure, einem partiellen Ester oder Vollester von Propylenglykol, Polyäthylenglykol oder eines Kohlenhydrats mit einer $C_2$-$C_{12}$-Fettsäure, einem pharmazeutischen verträglichen Ester oder Aether davon oder aus einem Gemisch der erwähnten Potentiatoren, und erwünschtenfalls herkömmliche therapeutisch inerte Hilfsstoffe für Suppositorien.

2. Suppositorien nach Anspruch 1, dadurch gekennzeichnet, dass der Wirkstoff Ceftriaxon-Dinatriumsalz-Trihydrat ist.

3. Suppositorien nach Anspruch 1 oder 2, dadurch gekennzeichnet, dass der Stabilisator aus einem Gemisch von Mono-, Di- und/oder Triglyceriden von $C_{12}$-$C_{18}$-Fettsäuren besteht.

4. Suppositorien nach Anspruch 3, dadurch gekennzeichnet, dass der Stabilisator vorwiegend aus dem Triglycerid der Laurinsäure ($C_{12}$) besteht.

5. Suppositorien nach einem der Ansprüche 1-4, dadurch gekennzeichnet, dass der Potentiator aus gesättigten $C_6$-$C_{12}$-Fettsäuren, ungesättigten $C_{16}$-$C_{18}$-Fettsäuren, Mono-, Di- oder Triglyceriden von $C_8$-$C_{12}$-Fettsäuren oder Mischungen davon, diese enthaltenden Speiseölen oder aus Mischungen davon besteht.

6. Suppositorien nach Anspruch 5, dadurch gekennzeichnet, dass der Potentiator aus einem Gemisch besteht, das vorwiegend Mono- und Diglyceride von $C_8-C_{10}$-Fettsäuren enthält.

7. Suppositorien nach Anspruch 6, dadurch gekennzeichnet, dass der Potentiator aus einem Gemisch besteht, das vorwiegend Mono- und Diglyceride der Capryl- und Caprinsäure enthält.

8. Suppositorien nach Anspruch 6 oder 7, dadurch gekennzeichnet, dass das Gemisch der Mono- und Diglyceride einen überwiegenden Anteil an Monoglycerid aufweist.

9. Suppositorien nach Anspruch 8, dadurch gekennzeichnet, dass der Anteil an Monoglycerid etwa 70% bis etwa 90% beträgt.

10. Suppositorien nach einem der Ansprüche 1-9, dadurch gekennzeichnet, dass die Menge an Ceftriaxon etwa 25 mg bis etwa 2000 mg beträgt.

11. Suppositorien nach Anspruch 10, dadurch gekennzeichnet, dass die Menge an Ceftriaxon etwa 50 bis etwa 500 mg beträgt.

12. Suppositorien nach einem der Ansprüche 1-11, dadurch gekennzeichnet, dass das Verhältnis Wirkstoff:Stabilisator zwischen etwa 1:32 und etwa 1:1 liegt.

13. Suppositorien nach Anspruch 12, dadurch gekennzeichnet, dass das Verhältnis Wirkstoff:Stabilisator zwischen etwa 1:5 und etwa 1:1,5 liegt.

14. Suppositorien nach einem der Ansprüche 1-13, dadurch gekennzeichnet, dass das Verhältnis Wirkstoff:Potentiator

0152896

zwischen etwa 1:32 und etwa 1:0,2 liegt.

15. Suppositorien nach Anspruch 14, dadurch gekennzeichnet, dass das Verhältnis Wirkstoff:Potentiator zwischen etwa 1:16 und 1:0,5 liegt.

16. Verfahren zur Herstellung von Suppositorien nach einem der Ansprüche 1-15, dadurch gekennzeichnet, dass man den Stabilisator zusammen mit dem Potentiator durch Erwärmen schmilzt, den Wirkstoff und erwünschtenfalls herkömmliche, therapeutisch inerte Hilfsstoffe für Suppositorien in der erhaltenen Schmelze gleichmässig dispergiert und die erhaltene Dispersion mittels Suppositorienformen entsprechender Grösse zu Suppositorien formuliert.

\*\*\*

Patentansprüche für Oesterreich

1. Verfahren zur Herstellung von Suppositorien, enthaltend als Wirkstoff eine therapeutisch wirksame Menge an Ceftriaxon, einem pharmazeutisch verträglichen Salz davon, oder einem Hydrat einer dieser Verbindungen, einen Stabilisator, bestehend aus einem Mono-, Di- oder Triglycerid einer $C_{12}$-$C_{18}$-Fettsäure oder aus einem Gemisch solcher Glyceride, einen Potentiator, bestehend aus einer aliphatischen $C_2$-$C_{18}$-Fettsäure, einem Mono-, Di- oder Triglycerid einer $C_2$-$C_{12}$-Fettsäure, einem partiellen Ester oder Vollester von Propylenglykol, Polyäthylenglykol oder eines Kohlenhydrats mit einer $C_2$-$C_{12}$-Fettsäure, einem pharmazeutischen verträglichen Ester oder Aether davon oder aus einem Gemisch der erwähnten Potentiatoren, und erwünschtenfalls herkömmliche therapeutisch inerte Hilfsstoffe für Suppositorien, dadurch gekennzeichnet, dass man den Stabilisator zusammen mit dem Potentiator durch Erwärmen schmilzt, den Wirkstoff und erwünschtenfalls herkömmliche, therapeutisch inerte Hilfsstoffe für Suppositorien in der erhaltenen Schmelze gleichmässig dispergiert und die erhaltene Dispersion mittels Suppositorienformen entsprechender Grösse zu Suppositorien formuliert.

2. Verfahren nach Anspruch 1, dadurch gekennzeichnet, dass der Wirkstoff Ceftriaxon-Dinatriumsalz-Trihydrat ist.

3. Verfahren nach Anspruch 1 oder 2, dadurch gekennzeichnet, dass der Stabilisator aus einem Gemisch von Mono-, Di- und/oder Triglyceriden von $C_{12}$-$C_{18}$-Fettsäuren besteht.

4. Verfahren nach Anspruch 3, dadurch gekennzeichnet, dass der Stabilisator vorwiegend aus dem Triglycerid der Laurinsäure ($C_{12}$) besteht.

5. Verfahren nach einem der Ansprüche 1-4, dadurch gekennzeichnet, dass der Potentiator aus gesättigten $C_6$-$C_{12}$-Fettsäuren, ungesättigten $C_{16}$-$C_{18}$-Fettsäuren, Mono-, Di- oder Triglyceriden von $C_8$-$C_{12}$-Fettsäuren oder Mischungen davon, diese enthaltenden Speiseölen oder aus Mischungen davon besteht.

6. Verfahren nach Anspruch 5, dadurch gekennzeichnet, dass der Potentiator aus einem Gemisch besteht, das vorwiegend Mono- und Diglyceride von $C_8$-$C_{10}$-Fettsäuren enthält.

7. Verfahren nach Anspruch 6, dadurch gekennzeichnet, dass der Potentiator aus einem Gemisch besteht, das vorwiegend Mono- und Diglyceride der Capryl- und Caprinsäure enthält.

8. Verfahren nach Anspruch 6 oder 7, dadurch gekennzeichnet, dass das Gemisch der Mono- und Diglyceride einen überwiegenden Anteil an Monoglycerid aufweist.

9. Verfahren nach Anspruch 8, dadurch gekennzeichnet, dass der Anteil an Monoglycerid etwa 70% bis etwa 90% beträgt.

10. Verfahren nach einem der Ansprüche 1-9, dadurch gekennzeichnet, dass die Menge an Ceftriaxon etwa 25 mg bis etwa 2000 mg beträgt.

11. Verfahren nach Anspruch 10, dadurch gekennzeichnet, dass die Menge an Ceftriaxon etwa 50 bis etwa 500 mg beträgt.

12. Verfahren nach einem der Ansprüche 1-11, dadurch gekennzeichnet, dass das Verhältnis Wirkstoff:Stabilisator zwischen etwa 1:32 und etwa 1:1 liegt.

13. Verfahren nach Anspruch 12, dadurch gekennzeichnet, dass das Verhältnis Wirkstoff:Stabilisator zwischen etwa 1:5

0152896

AT 4410/190

und etwa 1:1,5 liegt.

14. Verfahren nach einem der Ansprüche 1-13, dadurch gekennzeichnet, dass das Verhältnis Wirkstoff:Potentiator zwischen etwa 1:32 und etwa 1:0,2 liegt.

15. Verfahren nach Anspruch 14, dadurch gekennzeichnet, dass das Verhältnis Wirkstoff:Potentiator zwischen etwa 1:16 und 1:0,5 liegt.

***